# EUROPEAN PATENT APPLICATION

(11) **EP 4 501 314 A1**
(43) Date of publication of application: **05.02.2025**
(21) Application number: 23795163.7
(22) Date of filing: 19.04.2023
(51) Int. Cl.: A61K 9/08, A61K 31/135, A61K 31/137, A61K 9/00, A61K 47/18, A61P 25/24, A61P 25/22, A61K 9/12

(54) **R-KETAMINE LIQUID PREPARATION AND USE THEREOF**

(30) Priority: 26.04.2022 CN 202210448842
(71) Applicant: Yichang Humanwell Pharmaceutical Co., Ltd., Yichang, Hubei 443005 (CN)
(72) Inventor: WU, Youbin, Yichang, Hubei 443005 (CN); HUANG, Zhiyong, Yichang, Hubei 443005 (CN); WU, Jihong, Yichang, Hubei 443005 (CN); SHAO, Liangyu, Yichang, Hubei 443005 (CN); TIAN, Luanyuan, Yichang, Hubei 443005 (CN); ZHOU, Shimeng, Yichang, Hubei 443005 (CN); LIN, Xia, Yichang, Hubei 443005 (CN); XIANG, Yongyao, Yichang, Hubei 443005 (CN); LI, Xiaomei, Yichang, Hubei 443005 (CN); LV, Jinliang, Yichang, Hubei 443005 (CN); LI, Lie, Yichang, Hubei 443005 (CN); QU, Qin, Yichang, Hubei 443005 (CN)
(74) Representative: Modiano, Gabriella Diana
(86) International application number: PCT/CN2023/089329
(87) International publication number: WO 2023/207730

(57) **Abstract**

Disclosed in the present application is an R-ketamine liquid preparation. The R-ketamine liquid preparation is an aqueous solution preparation, and comprises R-ketamine or a pharmaceutically acceptable salt thereof, a pharmaceutically acceptable amphiphilic excipient, and water. The liquid preparation has a pH value of 2.5-5.7. The liquid preparation of the present application is administrated through oral mucosa, and features fast absorption, high bioavailability, simple administration, and good compliance. The preparation of the present application can be used for preventing, relieving, or treating major depression, unipolar depression, refractory depression, intractable depression, agitated depression, and bipolar depression.

## Description

This application claims priority to a Chinese patent application filed with the Chinese Patent Office on April 26, 2022, application number 202210448842.8 and the invention title "R-ketamine liquid preparation and use thereof", the contents of which should be understood to be incorporated herein by reference.

### Technical Field

The present application relates to, but is not limited to, the technical field of pharmaceutical formulations, and in particular to R-ketamine liquid formulations and their use as antidepressants.

### Background

Depression (Major depression disorder, MDD) is a common mental illness characterized by typical symptoms of low emotion, delayed thinking, and decreased and delayed speech movements. Depression seriously troubles patients' life and work, and brings a heavy burden to families and society. According to statistics, the number of suicide deaths caused by depression is estimated to be as high as 1 million every year. At present, antidepressants (such as tricyclic antidepressants, selective serotonin reuptake inhibitors, serotonin and norepinephrine reuptake inhibitors) are mainly used clinically to treat this mental illness. However, these drugs cannot play a therapeutic role for all patients with depression. Therefore, there is a need to develop novel drugs for treating depression.

In recent years, studies have proved that ketamine, as an antagonist of N-methyl-D-aspartate (NMDA) receptor, can increase the level of monoamine compounds in the brain by increasing the release of monoamine or inhibiting the reuptake of monoamine by presynaptic membrane, thus playing an antidepressant role. Therefore, this special psychiatric drug has attracted much attention.

In order to meet the needs of more patients with depression, reduce the cost of treatment, and reduce the burden on patients' families, it is urgent to develop a new liquid formulation with clinical application value.

### Summary

The present application provides an R-ketamine liquid formulation for oral mucosa administration. The formulation of the present application has high stability, rapid absorption, high bioavailability, simple application mode and good compliance.

In one aspect, the present application provides an R-ketamine liquid formulation for oral mucosa administration, comprising R-ketamine or a pharmaceutically acceptable salt thereof, an amphipathic pharmaceutically acceptable excipient, and water.

In another aspect, the present application provides a use of the aforementioned R-ketamine liquid formulation in the manufacture of a medicament for preventing, alleviating or treating depression, and the medicament can be used for treating major depression, unipolar depression, refractory depression, intractable depression, anxiety depression or bipolar depression.

In yet another aspect, the present application provides a method for preventing, alleviating or treating depression in a patient using the aforementioned R-ketamine liquid formulation, comprising administering to the patient a therapeutically effective amount of the R-ketamine liquid formulation.

In yet another aspect, the present application provides the aforementioned R-ketamine liquid formulation for use in preventing, alleviating or treating depression in a patient, and the depression is major depression, unipolar depression, refractory depression, intractable depression, anxiety depression or bipolar depression.

In yet another aspect, the present application provides a method for preparing the aforementioned R-ketamine liquid formulation.

### Brief Description of Drawings

The accompanying drawings are used to provide an understanding of the technical solutions of the present application, and constitute a part of the specification. They are used to explain the technical solutions of the present application together with the embodiments of the present application, and do not constitute a limitation to the technical solutions of the present application.
FIG. 1 is a diagram showing the in vitro diffusion of different R-ketamine formulations proposed in the present application.
FIG. 2 is a diagram of the relationship between administration routes and pharmacokinetics of different R-ketamine formulations proposed in the present application.
FIG. 3 is a diagram of the relationship between different administration routes and AUC of different R-ketamine formulations proposed in the present application.

### Detailed Description

A first aspect of the present application provides an R-ketamine liquid formulation for oral mucosa administration, comprising R-ketamine or a pharmaceutically acceptable salt thereof, an amphipathic pharmaceutically acceptable excipient, and water.

Unless otherwise specified, R-ketamine as used herein, also referred to as (R)-ketamine, arketamine, levoketamine, arketamine, d-ketamine, (2R)-2-(2-chlorophenyl)-2 (methylamino) cyclohexanone and the like, refers to the (R)-enantiomer of ketamine.

In the liquid formulation of the present application, whether R-ketamine or a pharmaceutically acceptable salt of R-ketamine is used, when calculating the content of active substance, the content of R-ketamine is calculated.

In one embodiment of the R-ketamine liquid formulation of the present application, the content of R-ketamine in the liquid formulation is 0.5% w/v - 8.5% w/v.

Since the R-ketamine liquid formulation of the present application has high bioavailability, the liquid formulation can be prepared as a product with a lower substance concentration, which cannot only meet the preventive or therapeutic needs of clinical patients, but also avoid the risk of abuse caused by high concentration administration. At the same time, R-ketamine liquid formulation has good compatibility with saliva, which reduces irritation to mucous membrane and reduces drug side effects.

In one embodiment, the concentration of R-ketamine in the liquid formulation is 0.7% w/v - 8.4% w/v. In another embodiment, the concentration of R-ketamine in the liquid formulation is 1.4% w/v - 6.3% w/v. In yet another embodiment, the concentration of R-ketamine in the liquid formulation is 1.4% w/v - 5.6% w/v. In still one embodiment, the concentration of R-ketamine in the liquid formulation is 0.7% w/v, 0.8% w/v, 0.9% w/v, 1.0% w/v, 1.1% w/v, 1.2% w/v, 1.3% w/v, 1.4% w/v, 1.5% w/v, 1.6% w/v, 1.7% w/v, 1.8% w/v, 1.9% w/v, 2.0% w/v, 2.1% w/v, 2.2% w/v, 2.3% w/v, 2.4% w/v, 2.5% w/v, 2.6% w/v, 2.7% w/v, 2.8% w/v, 2.9% w/v, 3.0% w/v, 3.1% w/v, 3.2% w/v, 3.3% w/v, 3.4% w/v, 3.5% w/v, 3.6% w/v, 3.7% w/v, 3.8% w/v, 3.9% w/v, 4.0% w/v, 4.1% w/v, 4.2% w/v, 4.3% w/v, 4.4% w/v, 4.5% w/v, 4.6% w/v, 4.7% w/v, 4.8% w/v, 4.9% w/v, 5.0% w/v, 5.1% w/v, 5.2% w/v, 5.3% w/v, 5.4% w/v, 5.5% w/v, 5.6% w/v, 5.7% w/v, 5.8% w/v, 5.9% w/v, 6.0% w/v, 6.1% w/v, 6.2% w/v, 6.3% w/v, 6.4% w/v, 6.5% w/v, 6.6% w/v, 6.7% w/v, 6.8% w/v, 6.9% w/v, 7.0% w/v, 7.1% w/v, 7.2% w/v, 7.3% w/v, 7.4% w/v, 7.5% w/v, 7.6% w/v, 7.7% w/v, 7.8% w/v, 7.9% w/v, 8.0% w/v, 8.1% w/v, 8.2% w/v, 8.3% w/v, or 8.4% w/v.

In another embodiment, acids that can form pharmaceutically acceptable salts with R-ketamine include hydrochloric acid, sulfuric acid, phosphoric acid, maleic acid, acetic acid, adipic acid, alginic acid, citric acid, aspartic acid, benzoic acid, benzenesulfonic acid, bisulfuric acid, butyric acid, camphoric acid, camphorsulfonic acid, glucaric acid, fumaric acid, glycerophosphoric acid, stearic acid, heptanoic acid, hexanoic acid, hydrobromic acid (i.e., HBr), hydroiodic acid (i.e., HI), lactic acid, methanesulfonic acid, nicotinic acid, oxalic acid, dihydroxynaphthoic acid, pectic acid, persulfuric acid, 3-phenylpropionic acid, picric acid, pivalic acid, propionic acid, succinic acid, tartaric acid, thiocyanic acid, glutamic acid, p-toluenesulfonic acid, undecanoic acid, and mandelic acid.

In one embodiment, pharmaceutically acceptable salts of R-ketamine include hydrochloride, sulfate, phosphate, maleate, acetate, adipate, alginate, citrate, aspartate, benzoate, benzenesulfonate, bisulfate, butyrate, camphorate, camphorsulfonate, glucarate, fumarate, glycerophosphate, stearate, heptanoate, hexanoate, salt of hydrobromic acid (i.e., HBr), salt of hydroiodic acid (i.e., HI), lactate, mesylate, nicotinate, oxalate, dihydroxynaphthate, pectate, persulfate, 3-phenylpropionate, picrate, pivalate, propionate, succinate, tartrate, thiocyanate, glutamate, bicarbonate, p-toluenesulfonate, undecanoate, and mandelate.

In one embodiment, the concentration of the amphiphilic pharmaceutically acceptable excipient in the liquid formulation is 0.03% w/v - 0.11% w/v. In another embodiment, the concentration of the amphiphilic pharmaceutically acceptable excipient in the liquid formulation is 0.03% w/v - 0.07% w/v. In still one embodiment, the concentration of the amphiphilic pharmaceutically acceptable excipient in the liquid formulation is 0.01% w/v, 0.013% w/v, 0.016% w/v, 0.02% w/v, 0.023% w/v, 0.026% w/v, 0.03% w/v, 0.033% w/v, 0.036% w/v, 0.04% w/v, 0.043% w/v, 0.046% w/v, 0.05% w/v, 0.053% w/v, 0.056% w/v, 0.06% w/v, 0.063% w/v, 0.066% w/v, 0.07% w/v, 0.073% w/v, 0.076% w/v, 0.08% w/v, 0.083% w/v, 0.086% w/v, 0.09% w/v, 0.093% w/v, 0.096% w/v, 0.10% w/v, 0.103% w/v, 0.106% w/v, or 0.11% w/v.

In one embodiment, the amphiphilic pharmaceutically acceptable excipient is one of sodium dodecyl sulfate, alkyl alcohol amide, alkyl succinate sulfonate, alcohol amine alkyl benzene sulfonate, naphthenate, sulfosuccinate, alkyl phenol sulfonate, polyoxyethylene monolaurate, sodium heptyl sulfate, sodium deoxycholate, sodium heptyl sulfonate, or a combination thereof, preferably sodium dodecyl sulfate, sodium deoxycholate.

In one embodiment, the liquid formulation has a pH of 2.5 - 5.7.

In another embodiment, the liquid formulation has a pH in the range of 3.0 - 5.7. In yet another embodiment, the R-ketamine liquid formulation has a pH in the range of 4.0- 5.7. In yet another embodiment, the liquid formulation has a pH in the range of 5.0 - 5.5. In still one embodiment, the liquid formulation has a pH of 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, or 5.7.

In another embodiment, the liquid formulation comprises R-ketamine or a pharmaceutically acceptable salt thereof with a concentration of 0.5% w/v - 8.5% w/v, an amphiphilic pharmaceutically acceptable excipient with a concentration of 0.01% w/v - 0.11% w/v, and water, and the liquid formulation has a pH in the range of 2.5 - 5.7.

In some embodiments of the present application, the R-ketamine liquid formulation may further comprise a tackifier.

In another embodiment, the tackifier is xanthan gum, sodium carboxymethylcellulose, hydroxypropyl methyl cellulose, methylcellulose, ethylcellulose, hydroxyethylcellulose, polyvinyl alcohol, carbomer, or polyvinylpyrrolidone, or a combination thereof.

In another embodiment, the sodium carboxymethyl cellulose is selected from one or more of sodium carboxymethyl cellulose 800, sodium carboxymethyl cellulose 4000, sodium carboxymethyl cellulose 8000, sodium carboxymethyl cellulose 12000.

In one embodiment, the combination of the tackifier comprises, without limitation, sodium carboxymethyl cellulose 4000 and sodium carboxymethyl cellulose 12000, sodium carboxymethyl cellulose 800 and sodium carboxymethyl cellulose 12000, sodium carboxymethyl cellulose 4000 and xanthan gum, sodium carboxymethyl cellulose 4000 and hydroxypropyl methyl cellulose, sodium carboxymethyl cellulose 4000 and xanthan gum composition, sodium carboxymethyl cellulose 4000 and hydroxypropyl methyl cellulose composition, and the ratio (w/w) of the combination of the two tackifiers is in the range of 1: 5 to 5: 1.

In one embodiment, the concentration of the tackifier is 0.01% w/v - 3.5% w/v. In another embodiment, the concentration of the tackifier is 0.05-3.0% w/v. In yet another embodiment, the concentration of the tackifier is 0.05-2.0% w/v. In still one embodiment, the concentration of the tackifier is 0.01 % w/v, 0.05 % w/v, 0.1 % w/v, 0.15 % w/v, 0.2 % w/v, 0.25 % w/v, 0.30 % w/v, 0.35 % w/v, 0.40 % w/v, 0.45 % w/v, 0.50 % w/v, 0.55 % w/v, 0.60 % w/v, 0.65 % w/v, 0.70 % w/v, 0.75 % w/v, 0.80 % w/v, 0.85 % w/v, 0.90 % w/v, 0.95 % w/v, or 1.00 % w/v.

In another embodiment, the R-ketamine liquid formulation further comprises one or more of the following auxiliary materials: a buffer, a flavoring agent, an antioxidant, an osmotic pressure regulator, and a preservative. In another embodiment, the R-ketamine liquid formulation further comprises one or more of the following auxiliary materials: a buffer, a flavoring agent, an antioxidant, an osmotic pressure regulator, a preservative, and a permeation enhancer.

In one embodiment of the R-ketamine liquid formulation of the present application, the buffer may be citric acid, sodium dihydrogen phosphate, disodium hydrogen phosphate, acetic acid, boric acid, sodium borate, succinic acid, tartaric acid, lactic acid, fumaric acid, trisodium phosphate (trisodium phosphate dodecahydrate or TSP), sodium benzoate, benzoic acid, sodium hydroxide, potassium hydroxide, alkali metal carbonate, sodium carbonate, imidazole, pyrophosphate, sodium gluconate, sodium lactate, phosphoric acid, borate, bicarbonate, Tris-HCl, citrate, or a combination thereof.

The buffer of the present application can maintain the pH of the liquid formulation within a stable range, which is beneficial to improve the stability of the liquid formulation. The concentration of the buffer is 0.1 - 0.3% w/v. In one embodiment, the concentration of the buffer is 0.1 - 0.2% w/v. In another embodiment, the concentration of the buffer is 0.1 - 0.15% w/v. In another embodiment, the concentration of the buffer is 0.1 % w/v, 0.11 % w/v, 0.12 % w/v, 0.13 % w/v, 0.14 % w/v, 0.15 % w/v, 0.16 % w/v, 0.17 % w/v, 0.18 % w/v, 0.19 % w/v, 0.2 % w/v, 0.21 % w/v, 0.22 % w/v, 0.23 % w/v, 0.24 % w/v, 0.25 % w/v, 0.26 % w/v, 0.27 % w/v, 0.28 % w/v, 0.29 % w/v, or 0.3 % w/v.

In another embodiment, the present application provides an R-ketamine liquid formulation, and the formulation further comprising a flavoring agent. The flavoring agent is capable of masking the off-flavor that a pharmaceutical composition may have and improving its palatability, which is beneficial to improving the compliance of a patient. In one embodiment, the flavoring agent of the liquid formulation is saccharin sodium, fructose, sucralose, stevioin, menthol, maltitol, xylitol, aspartame, cyclamate, saccharin, neohesperidin, thaumatin, stevia, or acesulfame, or a combination thereof.

In one embodiment, the present application provides an R-ketamine liquid formulation, wherein the concentration of the flavoring agent is 0.01-0.05% w/v. In another embodiment, the concentration of the flavoring agent is 0.01-0.03% w/v. In another embodiment, the concentration of the flavoring agent is 0.01% w/v, 0.02% w/v, 0.03% w/v, 0.04% w/v, or 0.05% w/v.

In one embodiment, the present application provides an R-ketamine liquid formulation, and the formulation further comprises an antioxidant. The antioxidant can effectively prevent or delay the oxidation of formulation, prevent the oxidative deterioration of drugs and their formulations, as well as problems such as discoloration and precipitation caused by oxidation, and increase the stability of drugs. In one embodiment, the antioxidant of the liquid formulation is ethylenediaminetetraacetic acid (EDTA) or a sodium or calcium salt thereof, vitamin E, gallate, sodium bisulfite, ascorbic acid or a salt thereof, butylhydroxyanisole or tocopherol, or a combination thereof.

In one embodiment, the present application provides an R-ketamine liquid formulation, and the concentration of the antioxidant is 0.010-0.020% w/v. In another embodiment, the concentration of the antioxidant is 0.010-0.015% w/v. In another embodiment, the concentration of the antioxidant of the liquid formulation is 0.010 % w/v, 0.011 % w/v, 0.012 % w/v, 0.013 % w/v, 0.014 % w/v, 0.015 % w/v, 0.016 % w/v, 0.017 % w/v, 0.018 % w/v, 0.019 % w/v, or 0.02 % w/v.

In one embodiment, the R-ketamine liquid formulation further comprises a preservative. In some embodiments, the preservative is selected from one or more of methyl paraben, ethyl paraben, propyl paraben, butyl paraben, sodium methyl paraben, sodium ethyl paraben, sodium propyl paraben, sodium butyl paraben, sorbic acid, potassium sorbate, sodium sorbate, benzoic acid, sodium benzoate, benzyl alcohol, benzalkonium bromide, benzalkonium chloride, trichlorotert-butanol, resorcinol, and sodium ethylenediamine tetraacetate.

In one embodiment, the present application provides an R-ketamine liquid formulation, and the concentration of the preservative is 0.010-0.020% w/v. In another embodiment, the concentration of the preservative is 0.010-0.015% w/v. In another embodiment, the concentration of the preservative of the liquid formulation is 0.010% w/v, 0.0125% w/v, 0.015% w/v, 0.0175% w/v or 0.02% w/v.

In one embodiment, the R-ketamine liquid formulation further comprises an osmotic pressure regulator. In some embodiments, the osmotic pressure regulator is selected from one or more of sodium chloride, potassium nitrate, boric acid, and glucose.

In one embodiment, the R-ketamine liquid formulation further comprises a permeation enhancer. In some embodiments, the permeation enhancer is one or more of propylene glycol, Tween 80, hydroxypropyl methyl cellulose, sodium dodecyl sulfate, sodium docusate, polysorbate, tetradecyl maltoside, lecithin, hydroxypropyl-β-cyclodextrin, sulfobutyl-β-cyclodextrin sodium, or PEG400.

A second aspect of the present application specifically provides an R-ketamine liquid formulation comprising R-ketamine hydrochloride, an amphiphilic pharmaceutically acceptable excipient, a tackifier, and water; the liquid formulation has a pH in the range of 4.5 - 5.5, and the content of R-ketamine in the liquid formulation is 0.7% w/v - 8.4% w/v.

In one embodiment, the kinds of tackifiers and their contents with amphiphilic pharmaceutically acceptable excipients are as previously described.

In another embodiment, the tackifier is one of sodium carboxymethyl cellulose 4000, sodium carboxymethyl cellulose 8000, sodium carboxymethyl cellulose 12000, xanthan gum, and hydroxypropyl methyl cellulose, or a combination thereof.

In the R-ketamine liquid formulation provided by the present application, the simultaneous use of an amphiphilic pharmaceutically acceptable excipient and a tackifier can make the API in the formulation more fully released in vitro, better promote the permeability of R-ketamine through oral mucosa, and synergistically improve the bioavailability of the R-ketamine liquid formulation.

In one embodiment of the R-ketamine liquid formulation of the present application, the tackifier is one of sodium carboxymethyl cellulose 4000, sodium carboxymethyl cellulose 8000, sodium carboxymethyl cellulose 12000, xanthan gum, and hydroxypropyl methyl cellulose, or a combination thereof. In one embodiment, the tackifier is xanthan gum. In another embodiment, the tackifier is hydroxypropyl methyl cellulose. In another embodiment, the tackifier is sodium carboxymethyl cellulose 4000 and sodium carboxymethyl cellulose 12000. In another embodiment, the tackifier is sodium carboxymethyl cellulose 4000 and hydroxypropyl methyl cellulose.

A third aspect of the present application specifically provides an R-ketamine liquid formulation comprising R-ketamine hydrochloride, an amphiphilic pharmaceutically acceptable excipient, a tackifier, a buffer, and water; the liquid formulation has a pH in the range of 4.5-5.5, and the content of R-ketamine in the liquid formulation is 0.7% w/v - 8.4% w/v.

In one embodiment, the kinds of tackifiers and buffers as well as their contents with amphiphilic pharmaceutically acceptable excipients are as previously described.

In one embodiment, the present application provides an R-ketamine liquid formulation in which the buffer is citric acid.

A fourth aspect of the present application specifically provides an R-ketamine liquid formulation comprising R-ketamine hydrochloride, an amphiphilic pharmaceutically acceptable excipient, a tackifier, an antioxidant, a flavoring agent, a buffer, and water; the liquid formulation has a pH in the range of 4.5 - 5.5, and the content of R-ketamine in the liquid formulation is 0.7% w/v - 8.4% w/v.

In one embodiment, the kinds of the tackifier, the antioxidant, the flavoring agent and the buffer as well as their contents with amphipathic pharmaceutically acceptable excipients are as previously described.

In another embodiment, the tackifier is one of sodium carboxymethyl cellulose 4000, sodium carboxymethyl cellulose 8000, sodium carboxymethyl cellulose 12000, xanthan gum, and hydroxypropyl methyl cellulose, or a combination thereof.

In one embodiment of the R-ketamine liquid formulation of the present application, the antioxidant is disodium edetate, the flavoring agent is sodium saccharin or maltitol, and the tackifier is one of sodium carboxymethyl cellulose 4000, sodium carboxymethyl cellulose 8000, sodium carboxymethyl cellulose 12000, xanthan gum, and hydroxypropyl methyl cellulose, or a combination thereof. In one embodiment, the antioxidant is disodium edetate, the flavoring agent is sodium saccharin or maltitol, and the tackifier is xanthan gum. In another embodiment, the antioxidant is disodium edetate, the flavoring agent is sodium saccharin or maltitol, and the tackifier is hydroxypropyl methyl cellulose. In another embodiment, the tackifier is sodium carboxymethyl cellulose 4000 and sodium carboxymethyl cellulose 12000. In another embodiment, the antioxidant is disodium edetate, the flavoring agent is sodium saccharin or maltitol, and the tackifier is sodium carboxymethyl cellulose 4000 and hydroxypropyl methyl cellulose.

In one embodiment, the present application provides an R-ketamine liquid formulation in which the buffer is citric acid.

The fifth aspect of the present application provides a use of an R-ketamine liquid formulation in the manufacture of a medicament for preventing, alleviating or treating depression, the medicament can be used for treating major depression, unipolar depression, refractory depression, intractable depression, anxiety depression, bipolar depression.

In one embodiment, the present application provides a use of an R-ketamine liquid formulation in the manufacture of a medicament for preventing, alleviating or treating depression, the liquid formulation is administered to a buccal membrane of the oral cavity of the patient.

A sixth aspect of the present application provides a method of preventing, alleviating or treating depression in a patient using the R-ketamine liquid formulation, comprising administering to the patient a therapeutically effective amount of the R-ketamine liquid formulation.

In one embodiment, the present application provides a method of preventing, alleviating or treating depression in a patient using the R-ketamine liquid formulation, the depression is major depression, unipolar depression, refractory depression, intractable depression, anxiety depression, bipolar depression.

In one embodiment, the present application provides a method of preventing, alleviating or treating depression in a patient using an R-ketamine liquid formulation, the liquid formulation is administered to a buccal membrane of the oral cavity of the patient.

A seventh aspect of the present application provides an R-ketamine liquid formulation for use in preventing, alleviating or treating depression in a patient, the depression is major depression, unipolar depression, refractory depression, intractable depression, anxiety depression, bipolar depression.

In one embodiment, an R-ketamine liquid formulation is for use in preventing, alleviating or treating depression in a patient, the liquid formulation is administered to the buccal membrane of the oral cavity of a mammal.

According to an eighth aspect, the present application provides a method for preparing the aforementioned R-ketamine liquid formulation, comprising the following preparation steps:
a) weighing a formulation amount of R-ketamine hydrochloride, adding purified water, fully stirring and dissolving, then sequentially adding a formulation amount of optional antioxidant such as disodium edetate, optional buffer such as citric acid, optional flavoring agent such as saccharin sodium, optional preservative such as benzalkonium chloride, and sufficiently stirring and dissolving;
b) adding formulation amounts of optional amphiphilic pharmaceutically acceptable excipient and optional tackifier, and sufficiently stirring and dissolving;
c) adjusting the pH to the corresponding value with sodium hydroxide;
d) transferring the solution to a specific volumetric flask, replenishing water to make the volume up to the mark; and
e) passing the solution through a 0.45 µm filter membrane after making the volume up to the mark, filling the filtrate into a prefilled syringe to obtain the finished product of the formulation.

The beneficial effects of the present application are as follows:
(1) The R-ketamine liquid formulation provided by the present application has rapid absorption, rapid onset of action within 3-7 minutes, and its AUC0-240min can be greater than 7000 ng/mL. The R-ketamine liquid formulation has high bioavailability, and at the same time, no crystallization phenomenon occurs, and the stability is high, which not only meets the needs of large-scale processing and production, but also reduces the medication risk of patients.
(2) The R-ketamine liquid formulation provided by the present application can effectively improve the bioavailability of the active substance by using the amphiphilic pharmaceutically acceptable excipient; the absorption amount of R-ketamine can be further improved by further adding a tackifier to increase the residence time of the liquid formulation in the oral mucosa, and the bioavailability of the R-ketamine liquid formulation can be synergistically improved.
(3) The administration method of the R-ketamine liquid formulation provided by the present application is simple and easy, and the patient compliance is greatly increased. Due to the small dosage and small volume, the irritation to the mucous membrane can be greatly reduced, and the risk of abuse can be reduced at the same time.

Concentrations of all ingredients are in weight/volume% (%w/v) unless otherwise specified. As is generally understood, the %w/v value refers to the amount of a particular component or ingredient in a formulation. It is well known that equivalent concentrations can be expressed in different units. For example, a concentration of 0.1% w/v can also be expressed as 1 mg/ml solution.

The present application discloses an R-ketamine liquid formulation for oral mucosa administration and use thereof, and those skilled in the art can learn from the contents of the present application and realize it by appropriately improving the process parameters. In particular, it should be pointed out that all similar substitutions and modifications will be apparent to those skilled in the art and are deemed to be included in the present application. The methods and applications of the present application have been described with reference to the preferred embodiments, and it will be apparent to those involved that the techniques of the present application will be realized and applied by making modifications or appropriate alterations and combinations of the methods and applications described herein without departing from the content, spirit and scope of the present application. The reagents or instruments used in the present application are commercially available.

### Instrument:

S220-K pH meter, SHJ-6AB water-bath kettle with magnetic stirrer, ME204T/02 electronic balance, ME3002T/02 electronic balance, DSC-800T fully-automatic transdermal diffusion instrument, Dionex U300 high performance liquid chromatograph.

### Example 1

### Formulation:

| | |
|---|---|
| R-ketamine hydrochloride | 64.6 mg |
| Citric acid | 1.5 mg |
| Disodium edetate | 0.12 mg |
| Sodium saccharin | 0.3 mg |
| Sodium carboxymethylcellulose 4000 | 1 mg |
| Sodium dodecyl sulfate | 0.66 mg |
| Benzalkonium chloride | 0.125 mg |
| Water | q.s. to 1 ml |

The preparation method comprises the following preparation steps:
a) weighing the formulation amount of R-ketamine hydrochloride, adding 80% of the total weight of purified water, fully stirring and dissolving, then sequentially adding the formulation amount of disodium edetate, citric acid, saccharin sodium and benzalkonium chloride, fully stirring, and dissolving;
b) adding the corresponding sodium dodecyl sulfate and sodium carboxymethyl cellulose 4000 in different formulcations, fully stirring, and dissolving;
c) adjusting the pH to 4.5-5.5 with sodium hydroxide;
d) transferring the solution to a specific volumetric flask, replenishing water to make the volume up to the mark of 10 ml; and
e) passing the solution through a 0.45 µm filter membrane after making the volume up to the mark, and filling the filtrate into a 1ml pre-filled syringe (purchased from Shandong Zibo Minkang Pharmaceutical Packaging Co., Ltd.), to obtain the finished product of the formulation in 1ml/piece.

### Example 2

### Formulation:

| | |
|---|---|
| R-ketamine hydrochloride | 64.6 mg |
| Citric acid | 1.5 mg |
| Disodium edetate | 0.12 mg |
| Sodium saccharin | 0.3 mg |
| Sodium carboxymethylcellulose 8000 | 1 mg |
| Sodium dodecyl sulfate | 0.66 mg |
| Benzalkonium chloride | 0.125 mg |
| Water | q.s. to 1 ml |

Example 1 was referred to for the preparation method.

### Example 3

### Formulation:

| | |
|---|---|
| R-ketamine hydrochloride | 64.6 mg |
| Citric acid | 1.5 mg |
| Disodium edetate | 0.12 mg |
| Sodium saccharin | 0.3 mg |
| Sodium carboxymethylcellulose 12000 | 1 mg |
| Sodium dodecyl sulfate | 0.66 mg |
| Benzalkonium chloride | 0.125 mg |
| Water | q.s. to 1 ml |

Example 1 was referred to for the preparation method.

### Example 4

### Formulation:

| | |
|---|---|
| R-ketamine hydrochloride | 64.6 mg |
| Citric acid | 1.5 mg |
| Disodium edetate | 0.12 mg |
| Sodium saccharin | 0.3 mg |
| Xanthan gum | 1 mg |
| Sodium dodecyl sulfate | 0.66 mg |
| Benzalkonium chloride | 0.125 mg |
| Water | q.s. to 1 ml |

Example 1 was referred to for the preparation method.

### Example 5

### Formulation:

| | |
|---|---|
| R-ketamine hydrochloride | 64.6 mg |
| Citric acid | 1.5 mg |
| Disodium edetate | 0.12 mg |
| Sodium saccharin | 0.3 mg |
| Sodium carboxymethylcellulose 4000 | 0.2 mg |
| Sodium carboxymethylcellulose 12000 | 0.8 mg |
| Sodium dodecyl sulfate | 0.66 mg |
| Benzalkonium chloride | 0.125 mg |
| Water | q.s. to 1 ml |

Example 1 was referred to for the preparation method.

### Example 6

### Formulation:

| | |
|---|---|
| R-ketamine hydrochloride | 64.6 mg |
| Citric acid | 1.5 mg |
| Disodium edetate | 0.12 mg |
| Sodium saccharin | 0.3 mg |
| Hydroxypropyl methyl cellulose | 1 mg |
| Sodium dodecyl sulfate | 0.66 mg |
| Benzalkonium chloride | 0.125 mg |
| Water | q.s. to 1 ml |

Example 1 was referred to for the preparation method.

### Example 7

### Formulation:

| | |
|---|---|
| R-ketamine hydrochloride | 64.6 mg |
| Citric acid | 1.5 mg |
| Disodium edetate | 0.12 mg |
| Sodium saccharin | 0.3 mg |
| Sodium carboxymethylcellulose 4000 | 0.2 mg |
| Hydroxypropyl methyl cellulose | 0.8 mg |
| Sodium dodecyl sulfate | 0.66 mg |
| Benzalkonium chloride | 0.125 mg |
| Water | q.s. to 1 ml |

Example 1 was referred to for the preparation method.

### Example 8

### Formulation:

| | |
|---|---|
| R-ketamine hydrochloride | 32.3 mg |
| Citric acid | 1.5 mg |
| Disodium edetate | 0.12 mg |
| Sodium saccharin | 0.3 mg |
| Sodium carboxymethylcellulose 4000 | 1 mg |
| Sodium dodecyl sulfate | 0.66 mg |
| Benzalkonium chloride | 0.125 mg |
| Water | q.s. to 1 ml |

Example 1 was referred to for the preparation method.

### Example 9

### Formulation:

| | |
|---|---|
| R-ketamine hydrochloride | 16.14 mg |
| Citric acid | 1.5 mg |
| Disodium edetate | 0.12 mg |
| Sodium saccharin | 0.3 mg |
| Sodium carboxymethylcellulose 4000 | 1 mg |
| Sodium dodecyl sulfate | 0.66 mg |
| Benzalkonium chloride | 0.125 mg |
| Water | q.s. to 1 ml |

Example 1 was referred to for the preparation method.

### Example 10

### Formulation:

| | |
|---|---|
| R-ketamine hydrochloride | 64.6 mg |
| Citric acid | 1.5 mg |
| Disodium edetate | 0.12 mg |
| Sodium saccharin | 0.3 mg |
| Sodium carboxymethylcellulose 4000 | 0.2 mg |
| Sodium dodecyl sulfate | 0.66 mg |
| Benzalkonium chloride | 0.125 mg |
| Water | q.s. to 1 ml |

Example 1 was referred to for the preparation method.

### Example 11

### Formulation:

| | |
|---|---|
| R-ketamine hydrochloride | 32.3 mg |
| Citric acid | 1.5 mg |
| Disodium edetate | 0.12 mg |
| Sodium saccharin | 0.3 mg |
| Sodium carboxymethylcellulose 4000 | 0.2 mg |
| Sodium dodecyl sulfate | 0.66 mg |
| Benzalkonium chloride | 0.125 mg |
| Water | q.s. to 1 ml |

Example 1 was referred to for the preparation method.

### Example 12

### Formulation:

| | |
|---|---|
| R-ketamine hydrochloride | 16.14 mg |
| Citric acid | 1.5 mg |
| Disodium edetate | 0.12 mg |
| Sodium saccharin | 0.3 mg |
| Sodium carboxymethylcellulose 4000 | 0.2 mg |
| Sodium dodecyl sulfate | 0.66 mg |
| Benzalkonium chloride | 0.125 mg |
| Water | q.s. to 1 ml |

Example 1 was referred to for the preparation method.

### Example 13

### Formulation:

| | |
|---|---|
| R-ketamine hydrochloride | 64.6 mg |
| Citric acid | 1.5 mg |
| Disodium edetate | 0.12 mg |
| Sodium saccharin | 0.3 mg |
| Sodium carboxymethylcellulose 4000 | 0.2 mg |
| Sodium alkyl succinate sulfonate | 0.66 mg |
| Benzalkonium chloride | 0.125 mg |
| Water | q.s. to 1 ml |

Example 1 was referred to for the preparation method.

Example 14 Stability test of R-ketamine hydrochloride buccal liquid of the present application

**Table 1 Impurity information of R-ketamine hydrochloride buccal liquid:**

| Name | Structural formula | Molecular formula and molecular weight | Chemical Name |
|---|---|---|---|
| Impurity B | 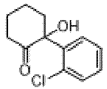 | C₁₂H₁₃O₂ | 2-(2-chlorophenyl)-2-(hydroxy) cyclohexanone |
| Impurity C | 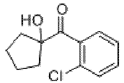 | C₁₂H₁₃ClO₂ | 1 -Hydroxycyclopentyl-2-chlorophenylmethanone |

The above two impurities can be purchased through commercial channels.

The present inventor(s) performed long-term stability experiments on the samples prepared in Examples 1 to 13 in an environment of 25°C± 2°C and RH 60% ± 5%.

### 1. Detection method of ketamine content:

### Determination according to high performance liquid chromatography (General Rule 0512)

Chromatographic conditions: octadecylsilane-bonded silica gel was used as a filler; 0.95 g of sodium hexane sulfonate was taken and dissolved in 1L of a mixed solution of acetonitrile and water (acetonitrile: water = 25: 75), 4 ml of glacial acetic acid was added and mixed uniformly to form a mobile phase. The detection wavelength was 215 nm. The flow rate was 1.0 ml per minute. The column temperature was 30°C. Injection volume was 20 µl.

### 2. Detection method of ketamine related substances:

Determination according to high performance liquid chromatography (General Rule 0512).

Chromatographic conditions: octadecylsilane-bonded silica gel was used as a filler (Agela MP C18 4.6 × 150 mm, 5 µm or equivalent column efficiency); 25 mM phosphate buffer solution (3.4 g of potassium dihydrogen phosphate was taken and dissolved in 1000 ml of water, and adjusted the pH value to 2.5 with phosphoric acid) was used as mobile phase A, and acetonitrile was used as mobile phase B. Gradient elution was performed according to the table below, and the detection wavelength was 215 nm. The column temperature was 30°C. The flow rate was 1.0 ml per minute. Injection volume was 20 µl.

**Table 2 Test conditions of raw auxiliary material content of detection samples**

| Time (minutes) | Mobile Phase A (%) | Mobile Phase B (%) |
|---|---|---|
| 0 | 85 | 15 |
| 35 | 60 | 40 |
| 37 | 60 | 40 |
| 65 | 20 | 80 |
| 70 | 20 | 80 |
| 71 | 85 | 15 |
| 75 | 85 | 15 |

**Table 3: Long-term test results of Examples 1-13**

| Formulation | Time (months) | pH | Content (%) | Related subs stances (%) | | | |
|---|---|---|---|---|---|---|---|
| | | | | Impurity B | Impurity C | Other individual impurity | Total impurities |
| Example 1 | 0 | 5.2 | 100.21 | Not detected | Not detected | Not detected | Not detected |
| | 3 | 5.1 | 100.68 | Not detected | Not detected | 0.011 | 0.016 |
| | 6 | 5.0 | 100.94 | Not detected | Not detected | 0.018 | 0.029 |
| Example 2 | 0 | 5.1 | 100.03 | Not detected | Not detected | Not detected | Not detected |
| | 3 | 5.1 | 100.36 | Not detected | Not detected | 0.016 | 0.025 |
| | 6 | 5.2 | 101.18 | Not detected | 0.01 | 0.034 | 0.049 |
| Example 3 | 0 | 5.1 | 99.78 | Not detected | Not detected | Not detected | Not detected |
| | 3 | 5.0 | 100.13 | Not detected | Not detected | 0.013 | 0.021 |
| | 6 | 4.9 | 101.22 | 0.01 | Not detected | 0.032 | 0.053 |
| Example 4 | 0 | 5.0 | 100.17 | Not detected | Not detected | Not detected | Not detected |
| | 3 | 5.0 | 101.09 | Not detected | Not detected | 0.01 | 0.017 |
| | 6 | 5.1 | 101.86 | 0.01 | 0.01 | 0.019 | 0.047 |
| Example 5 | 0 | 4.9 | 99.77 | Not detected | Not detected | Not detected | Not detected |
| | 3 | 5.0 | 100.35 | Not detected | 0.01 | 0.022 | 0.041 |
| | 6 | 5.2 | 101.34 | Not detected | 0.17 | 0.029 | 0.052 |
| Example 6 | 0 | 5.3 | 100.83 | Not detected | Not detected | Not detected | Not detected |
| | 3 | 5.2 | 101.41 | Not detected | Not detected | 0.024 | 0.033 |
| | 6 | 5.1 | 101.68 | 0.01 | 0.01 | 0.035 | 0.058 |
| Example 7 | 0 | 5.0 | 99.69 | Not detected | Not detected | Not detected | Not detected |
| | 3 | 5.1 | 100.12 | 0.01 | Not detected | 0.016 | 0.032 |
| | 6 | 5.3 | 100.56 | 0.014 | Not detected | 0.029 | 0.053 |
| Example 8 | 0 | 4.9 | 99.94 | Not detected | Not detected | Not detected | Not detected |
| | 3 | 5.0 | 101.05 | Not detected | 0.01 | 0.012 | 0.025 |
| | 6 | 5.2 | 102.33 | Not detected | 0.019 | 0.019 | 0.042 |
| Example 9 | 0 | 5.1 | 100.23 | Not detected | Not detected | Not detected | Not detected |
| | 3 | 5.3 | 100.64 | Not detected | Not detected | 0.018 | 0.025 |
| | 6 | 5.3 | 100.89 | Not detected | 0.01 | 0.031 | 0.050 |
| Example 10 | 0 | 5.0 | 98.76 | Not detected | Not detected | Not detected | Not detected |
| | 3 | 5.2 | 100.39 | Not detected | Not detected | 0.011 | 0.015 |
| | 6 | 5.3 | 101.03 | Not detected | Not detected | 0.022 | 0.034 |
| Example 11 | 0 | 4.8 | 99.68 | Not detected | Not detected | Not detected | Not detected |
| | 3 | 4.9 | 100.16 | Not detected | Not detected | 0.022 | 0.032 |
| | 6 | 5.0 | 100.72 | Not detected | 0.01 | 0.037 | 0.055 |
| Example 12 2 | 0 | 5.1 | 100.29 | Not detected | Not detected | Not detected | Not detected |
| | 3 | 5.1 | 101.34 | Not detected | Not detected | 0.034 | 0.040 |
| | 6 | 5.0 | 102.22 | 0.01 | Not detected | 0.051 | 0.068 |
| | 0 | 5.1 | 99.68 | Not detected | Not detected | Not detected | Not detected |
| Example 13 | 3 | 5.0 | 100.56 | Not detected | Not detected | 0.018 | 0.025 |
| | 6 | 5.0 | 101.23 | Not detected | 0.01 | 0.039 | 0.057 |

It can be seen from the results of the long-term test in Table 4 that the total impurities of R-ketamine hydrochloride buccal liquid are not more than 0.5%, and the maximum individual impurities are not more than 0.2% when the pharmaceutical formulation obtained in the present application is placed at room temperature for up to 6 months, and its quality still meets the quality standard, and the quality is stable.

### Example 15 In vitro permeation test of R-ketamine hydrochloride buccal liquid of the present application

In this study, the Franz diffusion cell method was used, and the phospholipid biomimetic barrier PermeaPad^{®} (biomimetic membrane) was selected to simulate the oral mucosa, and PBS buffer was used as the diffusion medium. After the diffusion cell reaches 37°C, 0.5 ml of artificial saliva was firstly added above the bionic membrane, and then 0.2 ml of formulation solution was added. The sampling time points were 10 min, 20 min, 30 min, 45 min, 60 min, 180 min, 240 min, 300 min, and 360 min, in waste mode, 1 ml sample was taken at each time point, and the content in liquid phase was detected.

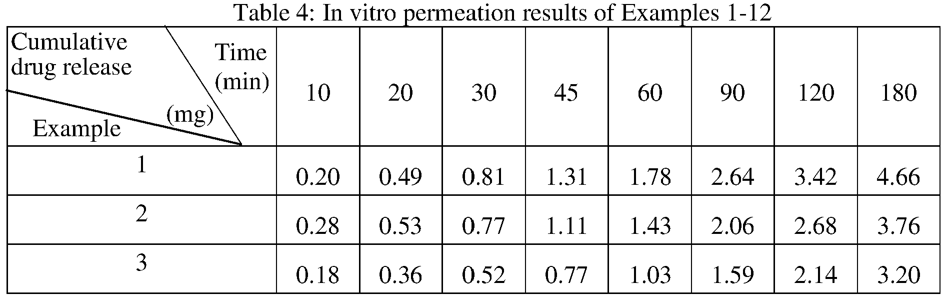

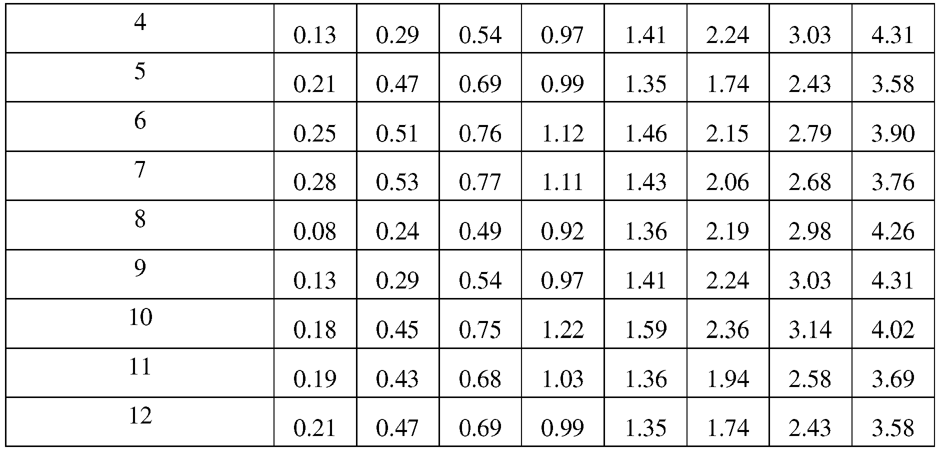

### Comparative Example 1 R-ketamine hydrochloride formulation for nasal administration (prepared with reference to patent CN 114306218 A)

### Components of the formulation

| | |
|---|---|
| R-ketamine hydrochloride | 3.228 g |
| Citric acid monohydrate | 0.03 g |
| Disodium edetate | 0.0036 g |
| Benzalkonium chloride | 0.0015 g |
| Sodium hydroxide | adjusted pH to 4.50 |
| Water | q.s. to 20.00 ml |

The preparation method comprises the following preparation steps:
a) weighing the formulation amount of R-ketamine hydrochloride, citric acid monohydrate, disodium edetate, benzalkonium chloride and 14g of purified water, fully stirring, and dissolving under the condition of 40 °C;
b) adjusting the pH to 4.5 with 1 M sodium hydroxide;
c) transferring the solution to a specific 20 ml volumetric flask, replenish water, and make the volume up to the mark; and
d) passing the solution through a 0.45 µm filter membrane after making the volume up to the mark, and filling the filtrate as needed.

### Comparative Example 2 R-ketamine hydrochloride formulation for nasal administration (prepared with reference to patent CN 114306218 A)

### Components of the formulation

| | |
|---|---|
| R-ketamine hydrochloride | 3.228 g |
| Citric acid monohydrate | 0.03 g |
| Disodium edetate | 0.0036 g |
| Benzalkonium chloride | 0.003 g |
| Sodium hydroxide | adjusted pH to 4.50 |
| Water | q.s. to 20.00 ml |

Comparative Example 1 was referred to for the preparation method.

### Comparative Example 3 R-ketamine hydrochloride formulation for nasal administration (prepared with reference to patent CN 114306218 A)

### Components of the formulation

| | |
|---|---|
| R-ketamine hydrochloride | 3.228 g |
| Citric acid monohydrate | 0.03 g |
| Disodium edetate | 0.0036 g |
| Benzalkonium chloride | 0.006 g |
| Sodium hydroxide | adjusted pH to 4.50 |
| Water | q.s. to 20.00 ml |

### Comparative Example 1 was referred to for the preparation method.

### Test Example 1 In vitro stability test

Example 14 was referred to for the experimental method.

**Table 5: Results of the stability test of Example 1 and Comparative Examples 1-3**

| Detection item | | Group | | | |
|---|---|---|---|---|---|
| | | Example 1 | Comparative Example 1 | Comparative Example 2 | Comparative Example 3 |
| Content | | 100.3% | 107.6% | 111.2% | 107.6% |
| Related substances | Maximum individual impurity | 0.09% | 0.09% | 0.09% | 0.09% |
| | Total impurities | 0.11% | 0.10% | 0.10% | 0.11% |
| pH value | | 4.99 | 4.53 | 4.52 | 4.51 |

It can be seen from the results of the stability test in Table 5 that after the stability test, the substance content, impurity content and pH value of the pharmaceutical formulation obtained in the present application all meet the standards. Comparative Examples 1-3 have abnormally increased substance content, indicating that the formulation is not highly stable.

### Test Example 2 Transdermal Diffusion Experiment in Vitro

In this study, the Franz diffusion cell method was used, and the phospholipid biomimetic barrier PermeaPad^{®} (biomimetic membrane) was selected to simulate the oral mucosa, and PBS buffer was used as the diffusion medium. After the diffusion cell reaches 37 °C, 0.5 ml of artificial saliva was firstly added above the bionic membrane, divided into groups, and then 0.2 ml of the solutions of Example 1, Comparative Example 1, Comparative Example 2 and Comparative Example 3 are added respectively. The sampling time points were 10 min, 20 min, 30 min, 45 min, 60 min, 180 min, 240 min, 300 min and 360 min. In waste mode, 1 ml sample was taken at each time point, and the content of liquid phase was detected.

**Table 6: In vitro permeation results of Example 1 and Comparative Examples 1-3**

| Time (min) | Example 1 | Comparative Example 1 | Comparative Example 2 | Comparative Example 3 |
|---|---|---|---|---|
| 10 | 3.59% | 3.77% | 3.34% | 3.13% |
| 20 | 6.02% | 6.34% | 5.71% | 5.48% |
| 30 | 8.35% | 8.50% | 7.40% | 7.27% |
| 45 | 11.91% | 11.45% | 9.43% | 9.61% |
| 60 | 15.58% | 14.16% | 11.15% | 11.69% |
| 90 | 22.63% | 19.19% | 14.18% | 15.76% |
| 120 | 27.07% | 21.81% | 15.17% | 17.86% |
| 180 | 40.56% | 31.72% | 21.13% | 26.51% |

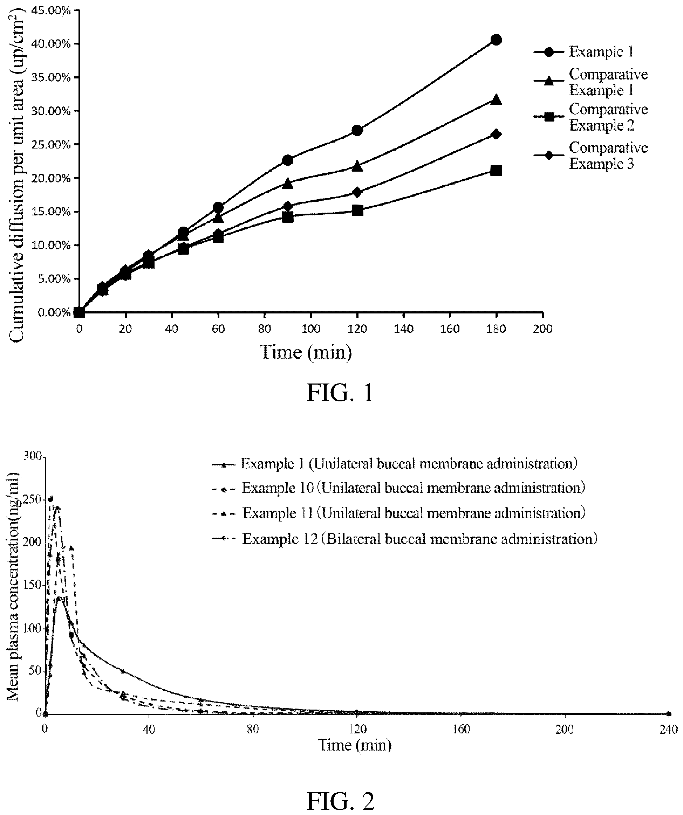

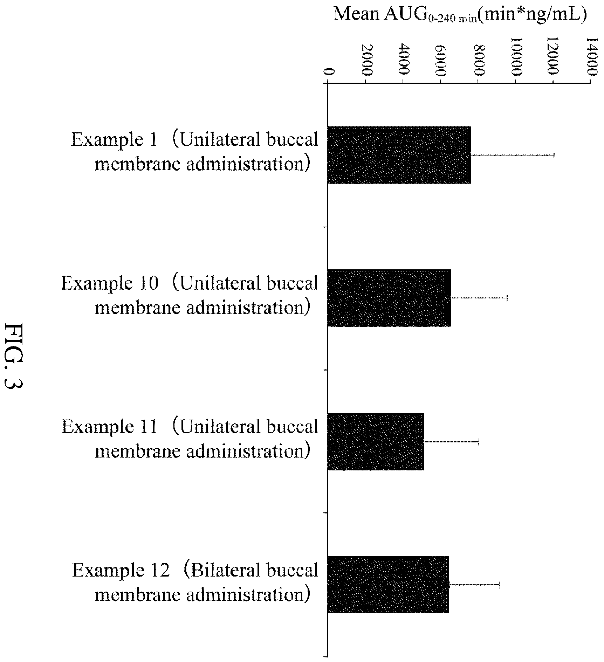

Specifically, the in vitro diffusion data of the four R-ketamine formulations are shown in FIG. 1. It can be seen from the above Table 6 and FIG. 3 that the R-ketamine hydrochloride buccal liquid prepared by the present invention has a higher transmucosal permeability per unit amount and a significantly improved release rate as compared with the formulation in which R-ketamine hydrochloride is absorbed through the nose in the Comparative Examples.

### Test Example 3 In vivo pharmacokinetic experiments

### 1. Experimental grouping

**Table 7: Grouping of animals**

| No. | Route of Administration |
|---|---|
| Example 1 | Unilateral buccal membrane administration |
| Example 10 | Unilateral buccal membrane administration |
| Example 11 | Unilateral buccal membrane administration |
| Example 12 | Bilateral buccal membrane administration |

### 2. Experimental protocol

In this experiment, New Zealand white rabbits, 2.5 kg - 3.0 kg, were used, 6 rabbits in each group, half male and half female. Four different formulations were administrated as R-ketamine hydrochloride buccal liquid at a dose of 7 mg/rabbit; the formulations of Examples 1 and 10 were administered as unilateral buccal liquid at a dose of 0.125 ml/animal; the formulation of Example 11 was administered as unilateral buccal liquid at a dose of 0.250 ml/ rabbit; the formulation of Example 12 was administered as bilateral buccal fluid at a dose of 0.250 ml/side. Before blood collection, the rabbits were placed in a rabbit fixed box. The blood collection method was ear vein blood collection. The blood collection time points were: 0 min (before administration), 2 min, 5 min, 10 min, 15 min, 30 min, 60 min, 120 min and 240 min. At each time point, about 1 mL of whole blood was transferred into a blood collection tube containing sodium heparin, centrifuged at 4000 rpm for 10 min. In centrifuged plasma samples, the supernatant was divided into 3 portions into 0.5 ml EP tube (the first 2 portions were filled with 150 µl, and the excess plasma was filled in the third cryopreservation tube as a backup sample), and then the plasma samples divided were stored at ≤-80 °C.

### 3. Experimental results

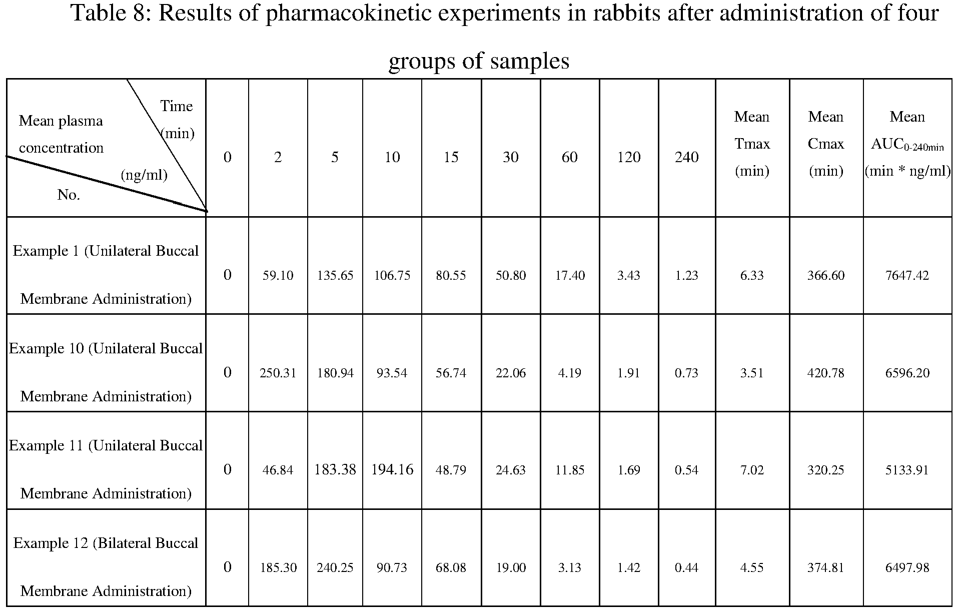

It can be seen from Table 8 and FIG. 2 and FIG. 3 that the plasma concentration of four different R-ketamine hydrochloride buccal liquids can reach the highest value within 3.5 - 7.1 minutes after buccal administration. The highest plasma concentration Cmax exceeded 320 ng/ml. AUC0-240min were all higher than 5000 min * ng/ml. It shows that the R-ketamine hydrochloride buccal liquid with different concentrations in the present application has fast absorption rate through buccal membrane administration, high plasma concentration in vivo, and can quickly exert drug efficacy in vivo.

The technical solution of the present application can also be used in a buccal liquid buccal membrane administration system of R-ketamine, ketamine or a pharmaceutically acceptable salt, and the object of the present application can be achieved by adjusting or changing the content of active ingredients, types and dosages of auxiliary materials that can be used in pharmacy, and the like.

Although the embodiments disclosed in the present application are as above, the contents described are only the embodiments adopted for the convenience of understanding the present application, and are not used to limit the present application. Any person skilled in the art to which this application belongs may make any modifications and changes in the form and details of implementation without departing from the idea and scope disclosed in this application, but the scope of protection of this application shall still be subject to the scope defined in the appended claims.

## Claims

1. An R-ketamine liquid formulation for oral mucosa administration, comprising R-ketamine or a pharmaceutically acceptable salt thereof, an amphiphilic pharmaceutically acceptable excipient, and water, wherein the liquid formulation has a pH in the range of 2.5 - 5.7.

2. The R-ketamine liquid formulation according to claim 1, wherein the liquid formulation comprises R-ketamine or a pharmaceutically acceptable salt thereof with a concentration of 0.5% w/v - 8.5% w/v, an amphiphilic pharmaceutically acceptable excipient with a concentration of 0.01% w/v - 0.11% w/v, and water, and the liquid formulation has a pH in the range of 2.5 - 5.7.

3. The R-ketamine liquid formulation according to claim 2, wherein the concentration of R-ketamine in the liquid formulation is 0.7% w/v - 8.4% w/v; preferably 1.4% w/v - 6.3% w/v; more preferably 1.4% w/v - 5.6% w/v.

4. The R-ketamine liquid formulation according to claim 2, wherein the amphiphilic pharmaceutically acceptable excipient in the liquid formulation is one of sodium dodecyl sulfate, alkyl alcohol amide, alkyl succinate sulfonate, alcohol amine alkyl benzene sulfonate, naphthenate, sulfosuccinate, alkyl phenol sulfonate, polyoxyethylene monolaurate, sodium heptyl sulfate, sodium deoxycholate, sodium heptyl sulfonate, or a combination thereof, preferably sodium dodecyl sulfate, sodium deoxycholate.

5. The R-ketamine liquid formulation according to claim 2, wherein the concentration of the amphiphilic pharmaceutically acceptable excipient in the liquid formulation is 0.01% w/v - 0.11% w/v; preferably 0.03% w/v - 0.09% w/v; and more preferably 0.05% w/v - 0.07% w/v.

6. The R-ketamine liquid formulation according to claim 2, wherein the liquid formulation has a pH in the range of 3.0 - 5.7; preferably 4.0-5.7; more preferably 4.5 - 5.5; and particularly preferably 5.0 - 5.5.

7. The R-ketamine liquid formulation according to any one of claims 1 to 6, wherein the liquid formulation further comprises a tackifier.

8. The R-ketamine liquid formulation according to claim 7, wherein the tackifier is one of xanthan gum, sodium carboxymethyl cellulose, hydroxypropyl methyl cellulose, methyl cellulose, ethyl cellulose, hydroxyethyl cellulose, polyvinyl alcohol, carbomer, and polyvinylpyrrolidone, or a combination thereof.

9. The R-ketamine liquid formulation according to claim 8, wherein the sodium carboxymethyl cellulose is one of sodium carboxymethyl cellulose 800, sodium carboxymethyl cellulose 4000, sodium carboxymethyl cellulose 8000, or sodium carboxymethyl cellulose 12000, or a combination thereof.

10. The R-ketamine liquid formulation according to claim 7, wherein the concentration of the tackifier is 0.01% w/v - 3.5% w/v.

11. The R-ketamine liquid formulation according to any one of claims 1 to 10, wherein the liquid formulation further comprises one or more of the following auxiliary materials: a buffer, a flavoring agent, an antioxidant, an osmotic pressure regulator, and a preservative; or, the liquid formulation further comprises one or more of the following auxiliary materials: a buffer, a flavoring agent, an antioxidant, an osmotic pressure regulator, a preservative, and a permeation enhancer.

12. The R-ketamine liquid formulation according to claim 11, wherein the liquid formulation further comprises a buffer, a flavoring agent, an antioxidant, and a preservative.

13. The R-ketamine liquid formulation according to claim 11 or 12, wherein the buffer is one of citric acid, sodium citrate, acetic acid, sodium acetate, lactic acid, sodium dihydrogen phosphate, disodium hydrogen phosphate, succinic acid, boric acid, sodium borate, tartaric acid, and fumaric acid, or a combination thereof.

14. The R-ketamine liquid formulation according to claim 11 or 12, wherein the flavoring agent is one of saccharin sodium, fructose, sucralose, stevioin, menthol, maltitol, xylitol, aspartame, cyclamate, saccharin, neohesperidin, thaumatin, stevia, and acesulfame, or a combination thereof.

15. The R-ketamine liquid formulation according to claim 11 or 12, wherein the antioxidant is one of disodium edetate, vitamin E, gallate, sodium bisulfite, ascorbic acid or a salt thereof, butylhydroxyanisole, and tocopherol, or a combination thereof.

16. The R-ketamine liquid formulation according to claim 11 or 12, wherein the preservative is one of methyl hydroxybenzoate, ethyl paraben, propyl paraben, butyl paraben, sodium methyl paraben, sodium ethyl paraben, sodium propyl paraben, sodium butyl paraben, sorbic acid, potassium sorbate, sodium sorbate, benzoic acid, sodium benzoate, benzyl alcohol, benzalkonium bromide, benzalkonium chloride, trichlorobutanol, resorcinol, and sodium ethylenediamine tetraacetate, or a combination thereof.

17. Use of the R-ketamine liquid formulation according to any one of claims 1 to 16 in the manufacture of a medicament for preventing, alleviating or treating depression.

18. The use according to claim 17, wherein the depression is major depression, unipolar depression, refractory depression, intractable depression, anxiety depression, or bipolar depression.

19. The use according to claim 17 or 18, wherein the liquid formulation is administered to a buccal membrane of the oral cavity of a patient.

20. A method for preventing, alleviating or treating depression in a patient with the R-ketamine liquid formulation according to any one of claims 1 to 16, comprising administering a therapeutically effective amount of the R-ketamine liquid formulation to the buccal membrane of the oral cavity of the patient.

21. The method according to claim 20, wherein the depression is major depression, unipolar depression, refractory depression, intractable depression, anxiety depression, or bipolar depression.

22. The R-ketamine liquid formulation according to any one of claims 1 to 16, for use in preventing, alleviating or treating depression in a patient.

23. The R-ketamine liquid formulation according to claim 22, for use in preventing, alleviating or treating depression in a patient, wherein the depression is major depression, unipolar depression, refractory depression, intractable depression, anxiety depression, or bipolar depression.

24. The R-ketamine liquid formulation according to claim 22 or 23, for use in preventing, alleviating or treating depression in a patient, wherein the liquid formulation is administered to a buccal membrane of the oral cavity of the patient.
